# EUROPEAN PATENT APPLICATION

(11) **EP 2 052 679 A1**
(43) Date of publication of application: **29.04.2009**
(21) Application number: 08018080.5
(22) Date of filing: 15.10.2008
(51) Int. Cl.: A61B 5/026, A61B 5/0476

(54) **Electrophysiological assessment of the integrity of cerebral vasculature**

(30) Priority: 24.10.2007 US 977272
(71) Applicant: Medametrics LLC, Cupertino CA 95014 (US)
(72) Inventor: Rewari, Gaurav, Cupertino CA 95014 (US)
(74) Representative: Meissner, Bolte & Partner

(57) **Abstract**

A method for monitoring blood flow in at least one region of cerebral vasculature of a subject, the method comprising determining an electrophysiological activity in the at least one region of the cerebral vasculature using a non-invasive technique; determining a deviation of the electrophysiological activity from a baseline of electrophysiological activity attributed to the at least one region of the cerebral vasculature; and determining the blood flow in specific branches of the at least one region of the cerebral vasculature based on the deviation.

## Description

### BACKGROUND

The present invention relates to the field of monitoring blood flow in cerebral vasculature. More specifically, it relates to a method for monitoring blood flow in cerebral vasculature by using electromagnetic means to assess the integrity of the cerebral vasculature.

The brain is a critical organ of the human body and comprises neurons that have a high rate of metabolic activity. Therefore, it needs a regular supply of nutrients and oxygen. In the human body, blood supplied through the cerebral vasculature is the only source of nutrients and oxygen to the neurons in the brain. Accordingly, though the brain represents around two percent of the total body weight, it receives almost one-fifth of total blood output from the heart.

An interruption in the blood supply to parts of the brain may result in an injury to the neurons in the brain. The blood supply may be interrupted due to an occlusion and/or hemorrhage in a blood vessel in the cerebral vasculature. The injury to the neurons is also referred to as a stroke or cerebrovascular accident (CVA). A stroke may result in disabilities such as paralysis, cognitive deficits, speech impairment, and in extreme conditions, coma and even death. Hence, it is critical to monitor the blood supply to the brain during neurological diagnostics, during and after neurosurgical procedures, and for post-trauma patient monitoring to monitor the integrity of the cerebral vasculature.

Further, the blood supply to the brain needs to be continuously monitored for patients suffering from aneurysms. For some days after the first incidence of aneurysm, the cerebral vasculature may go into vasospasms, causing secondary strokes that may be fatal. These secondary strokes are idiosyncratic, multi-focal and sudden, and often go undetected. The timely detection of secondary strokes may help a physician to take the necessary corrective steps.

Various techniques are used to monitor the blood supply to the brain by monitoring the blood flow in the cerebral vasculature. One of the primary techniques includes Magnetic Resonance Imaging (MRI). The technique uses a strong magnetic field to align the hydrogen atoms present in the tissue to be imaged, and then delivers a strong electromagnetic pulse to excite them. As the pulse is turned off, the hydrogen atoms revert to their original state. The time required for the hydrogen atoms to return to the original state depends on their number and the characteristic physical properties of the tissue. This time is measured and used to construct images of the tissue. Magnetic Resonance Angiography (MRA) and functional Magnetic Resonance Imaging (fMRI) are two variations of the MRI technique, which are used to assess cerebral blood flow.

MRA involves administering a magnetic contrast media in the blood stream of a subject. As it flows through the blood, the path of this contrast media is traced and used to check the integrity of the blood vessels.

The fMRI technique detects alterations in the blood flow to the affected areas of the brain. This technique is based on the fact that the magnetic properties of oxygenated blood and deoxygenated blood are different. The magnetic resonance (MR) signal of the blood varies with its oxygen level. Thus, variation in the oxygen level of the blood can be detected by providing a suitable MR pulse sequence in an MRI scanner. The response to the MR pulse sequence is used to construct the image that shows the oxygen level in the affected areas of the brain. This oxygen level, in turn, indicates the blood flow in those areas.

Positron Emission Tomography (PET) is another technique used to determine the blood flow in the brain. It includes administering a positron-emitting radioactive isotope, which is incorporated within metabolically active molecules such as fluorodeoxyglucose (FDG), to a patient. Each positron that is emitted from the isotope immediately interacts with an electron in the human body to generate two photons of equal energy traveling in opposite directions. The simultaneous arrival of these two photons is detected by using detectors that are placed circumferentially around the patient. The line of propagation of the two photons is used to identify their source location. Hence, the path of the radioactive isotope, incorporated within the metabolically active molecule, is tracked as it flows through the blood stream. The number of photons coming from a particular region is related to the blood flow in that region, and hence, facilitates the process of determining the blood flow.

Another method used to measure cerebral blood flow is Transcranial Doppler Ultrasonography (TCD). This method uses the principle of the Doppler shift, in which the frequency of sound waves is altered when they are reflected from a moving target. In this method, ultrasonic waves are directed to the area of the brain that is being investigated. A change is observed in the frequency of the ultrasonic waves when they reflect from the red blood cells. This change is based on the velocity of the moving red blood cells. Therefore, the change in the frequency provides an indication of the blood flow.

Diffused Optical Tomography (DOT) is another method, which uses near infrared light to image brain tissues. This technique measures variations in the optical absorption of hemoglobin, which varies with the level of oxygen in the blood. An array of light sources and detectors is placed over the skull. The near infrared light from the light sources penetrates the skull to a certain depth, beyond which it is completely scattered. The complex pattern of light emerging from the brain tissue is detected by the detectors and is converted into electrical signals. Thereafter, a computer processes these signals to reconstruct an image of the brain tissue, based on the level of oxygen in the blood. The level of oxygen in the tissue gives an estimate of the blood flow in the area.

Another method uses implanted electrodes to measure the electrical activity in the cerebral cortex. Any change in the blood flow to the brain is reflected in the altered electrical activity in the brain, and hence, can be detected. This invasive measurement technique is known as electrocorticography. It is used in intra-operative conditions to infer the overall status of the blood flow in the brain under the influence of anesthesia.

Early detection of vasospasm after acute subarachnoid hemorrhage by using continuous monitoring by Electroencephalogram (EEG) has been reported by Vespa, P.M., Nuwer, M.R., Juhász, C., Alexander, M., Nenov, V., Martin, N., and Becker, D.P. in the journal, Electroencephalography and Clinical Neurophysiology, Volume 103, Issue 6, December 1997, Pages 607-615. The method involves predicting vasospasm based on the relative measurements carried out on the EEG recordings. The method provides the overall status of the integrity of the cerebral vasculature.

However, the methods and systems described in the prior art suffer from one or more of the following drawbacks. Firstly, prior art methods follow invasive procedures. Further, they are only used during intra-operative procedures. The methods indicate the overall status of the blood flow in the cerebral vasculature, not in specific branches. Further, these systems require bulky instrumentation and are therefore not generally suited for bedside monitoring. Moreover, the period during which a patient can be monitored continuously is limited. During scanning, the patient is generally secured on a platform to avoid motion artifacts, and is then moved inside the scanning tunnel. This may create uneasiness in some patients due to claustrophobia. Moreover, implementation of these methods involves high instrumentation and operational costs.

Hence, there is a need for a method that can be used to continuously monitor cerebral blood flow. The implementation of the method should reduce the amount of bulky instrumentation required, as well as operational costs. It should also be suitable for bedside monitoring. Further, the method should be non-invasive and usable in non-operative conditions. It should also provide status of the blood flow in specific branches of the cerebral vasculature.

### SUMMARY

The present invention provides a method for monitoring the blood flow in specific branches of the cerebral vasculature of a subject.

An object of the present invention is to provide a non-invasive method for continuous monitoring of the blood flow in specific branches of the cerebral vasculature.

Another object of the present invention is to provide a method for determining the blood flow in specific branches of the cerebral vasculature.

Another object of the present invention is to provide a method that is suitable for continuous bedside monitoring of the subject.

The human brain comprises a large number of neurons. The electrophysiological activity of these neurons is closely associated with changes in the blood flow and the blood oxygen level around them. Any change in the blood flow to a region of the brain results in altered electrophysiological activity in the region. Accordingly, the present invention provides a method for monitoring the blood flow in one or more regions of the cerebral vasculature by monitoring the electrophysiological activity in one or more corresponding regions of the brain. The method involves determining the electrophysiological activity of at least one region of the brain by using a non-invasive technique. This electrophysiological activity is then compared with a first baseline of the electrophysiological activity in the at least one region. The blood flow in this specific region of the brain is then determined, based on the deviation between the measured electrophysiological activity and the first baseline.

In an embodiment of the present invention, the method includes exciting at least one region of the brain and measuring the corresponding electrophysiological activity of the region. In an embodiment of the present invention, the at least one region of the brain is excited by applying specific external stimuli to a subject. In another embodiment of the present invention, the at least one region of the brain is excited by inducing the subject to perform specific tasks. The electrophysiological activity is then compared with a second baseline of the electrophysiological activity of the at least one region. Any deviation in the measured electrophysiological activity from the second baseline signifies an altered blood flow to the at least one region. Each region in the brain receives blood through specific branches of the cerebral vasculature. Hence, based on the region of the brain that has altered electrophysiological activity, the specific branch of the cerebral vasculature with the altered blood flow can be determined.

The present invention obviates the need for bulky instrumentation and reduces the operational costs involved. It is therefore suitable for continuous bedside monitoring of the blood flow in the cerebral vasculature. The present invention may be implemented in operative as well as non-operative conditions.

### BRIEF DESCRIPTION OF THE DRAWINGS

Various embodiments of the present invention will hereinafter be described in conjunction with the appended drawings, provided to illustrate and not to limit the present invention, wherein like designations denote like elements, and in which:
FIG. 1 is a block diagram depicting a cerebral vasculature monitoring system, in accordance with an embodiment of the present invention;
FIG. 2 is a flowchart depicting a method for monitoring the blood flow in different regions of the cerebral vasculature, in accordance with an embodiment of the present invention; and
FIG. 3 is a flowchart depicting a method for monitoring the blood flow in different regions of the cerebral vasculature, in accordance with another embodiment of the present invention.

### DETAILED DESCRIPTION OF THE INVENTION

The present invention relates to a method for monitoring the blood flow in the cerebral vasculature of a subject by measuring the electrophysiological activity of the neurons in the brain. Electrophysiological activity is the electrical activity caused by movements of ions across biological tissues.

The electrophysiological activity of the neurons is closely associated with the changes in the blood flow and blood oxygen level around them. Hence, a change in the blood flow to the neurons results in altered neuronal activity, which is manifested in the altered electrophysiological activity of the neurons. The present invention provides a method for determining the blood flow in the cerebral vasculature by detecting the altered electrophysiological activity, and thereafter correlating this alteration with the change in the blood flow.

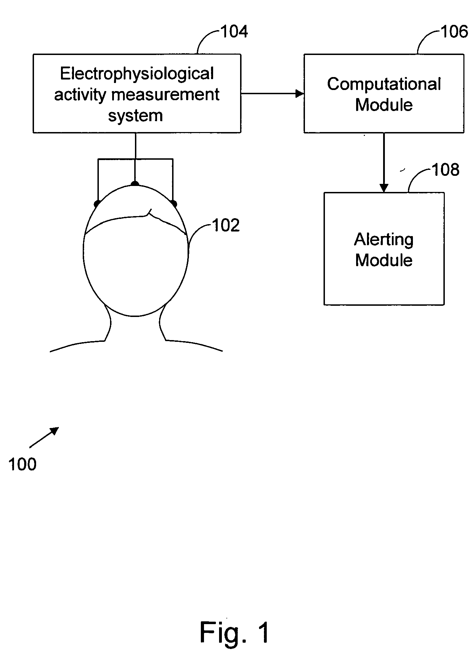

FIG. 1 is a block diagram depicting a Cerebral Vasculature Monitoring System (CVMS) 100. CVMS 100 comprises an electrophysiological activity-measurement system 104, a computational module 106, and an alerting module 108. Various embodiments of CVMS 100 monitor the integrity of the cerebral vasculature of a subject 102.

The present invention proposes a method for monitoring the blood flow in the cerebral vasculature of subject 102. According to the method, electrophysiological activity measurement system 104 measures the electrophysiological activity of specific regions of the brain of subject 102. Electrophysiological activity measurement system 104 provides the measured electrophysiological activity to computational module 106. Computational module 106 compares the measured electrophysiological activity with a pre-established baseline of the electrophysiological activity of the respective regions of the cerebral vasculature of subject 102. The pre-established baseline corresponds to the normal blood flow in the cerebral vasculature of the particular region of the brain. Computational module 106 determines the blood flow in the specific region of the brain from the pre-established baseline, based on the deviation of the measured electrophysiological activity. If the blood flow in the specific branches of the cerebral vasculature is altered, alerting module 108 generates an alert.

Various examples of electrophysiological activity-measurement system 104 include, but are not limited to, the electroencephalography (EEG) system and the magnetoencephalography (MEG) system. EEG and MEG are non-invasive measurement techniques and do not require a medical procedure to penetrate or break the skin or a body cavity to take the measurements.

Electroencephalography refers to the measurement of the electrical activity in the brain, which is generally recorded through electrodes placed on the scalp. A graphic record of this measured electrical activity is known as an electroencephalogram. The electrical activity of the brain varies with time, creating various patterns that can be seen in an electroencephalogram. These patterns represent electrical signals from a large number of neurons.

Magnetoencephalography measures the magnetic fields produced by the electrical activity of the brain. This measurement is taken by picking up magnetic signals outside the scalp by using sensitive magnetic field detectors. The measured signal is then processed by using inversion algorithms to approximate the location of the source of the electrical activity in the brain.

Computational module 106 compares the measured electrophysiological activity associated with each region of the cerebral vasculature with a corresponding pre-established baseline. Further, computational module 106 determines the change in the blood flow in the different branches of the cerebral vasculature in the particular region. This change in blood flow is determined, based on the degree of deviation between the measured electrophysiological activity and the corresponding pre-established baseline. In an embodiment of the present invention, the degree of deviation can be determined by calculating the relative difference between the amplitudes of the measured electrophysiological activity and the amplitudes of the baselines. The relative difference between the amplitudes can be expressed as a percentage of the corresponding amplitude of the baseline. In an embodiment of the present invention, the calculation can be carried out in the time domain. In an alternative embodiment of the present invention, the calculation can be carried out in the frequency domain. The measured electrophysiological activity is considered to be altered if the calculated deviation from the baseline exceeds a pre-defined threshold. When the altered electrophysiological activity is detected, a signal is sent to alerting module 108 to generate an alert.

The electrophysiological activity of the brain is monitored under normal as well as excited conditions of the brain for a large sample population. The empirical data thus generated is analyzed to establish patterns associated with the activities of a region of the brain during normal and excited conditions of the brain. The patterns are stored in computational module 106, to serve as baselines of electrophysiological activity during the corresponding set of conditions.

Baselines that correspond to the normal activity of the unexcited regions of the brain will be hereinafter referred to as first baselines. The measured electrophysiological activity of an unexcited region of the brain is compared with a first baseline corresponding to the unexcited region of the brain. The first baseline may be either latitudinal or individual. A latitudinal first baseline of each region of the brain can be established by performing one or more measurements of the electrophysiological activity of each region of the brain of each subject in a population, and averaging the measurements. Alternatively, an individual first baseline may also be established for each subject by averaging the multiple measurements taken of the subject while in a normal condition. In an embodiment of the present invention, the normal condition of the subject signifies that the subject is in a healthy state. In an embodiment of the present invention, the individual first baseline is preferred over the latitudinal first baseline, to generate accurate results. In situations where both such the baselines are unavailable, a quick initial measurement of the electrophysiological activity of the subject's brain can serve as the first baseline.

In another embodiment of the present invention, the assessment of the blood flow in specific regions of the brain is performed by selectively exciting specific regions of the brain. It is known that different regions of the brain respond to different types of stimuli. Hence, a specific region can be excited by providing a particular type of external stimulus. Since the activity of each region, in response to external stimuli, differs from its normal activity, the baseline to be used for the comparison needs to be established for the activity of each region, in response to a specific external stimulus. Such a baseline will hereinafter be referred to as a second baseline. The measured electrophysiological activity of an excited region of the brain is compared with the second baseline corresponding to the excited region of the brain. A latitudinal second baseline and an individual second baseline can be established in a similar manner, as described in the context of the first baseline.

The electrophysiological activity of a region of the brain may deviate from the normal activity, even when no external stimulus is provided. Such an activity is referred to as a spontaneous activity. In an embodiment of the present invention, the electrophysiological activity of a region of the brain is measured and compared with the first baseline of that region. If the region exhibits spontaneous activity, the measured activity deviates from the first baseline of that region. If this deviation exceeds a certain threshold, an alert is generated to indicate altered activity. Thus, this method can be used to monitor spontaneous activities such as epileptic episodes, and the like.

A correlation between the baselines and the actual blood flow in the cerebral vasculature needs to be established. In an embodiment of the present invention, the measurement of the blood flow in specific branches of the cerebral vasculature, by using indirect measurement techniques such as fMRI or MEG, can be carried out while establishing the baselines. This simultaneous measurement can be used to determine the exact correlation between the baselines and the blood flow.

The normal electrophysiological activity of the brain may vary among individuals belonging to different age groups, genders and races, and other such parameters.

The normal electrophysiological activity of the brain can also be influenced by the medical condition of an individual. These variations need to be accounted for while establishing the first and second latitudinal baselines. Accordingly, in an embodiment of the present invention, latitudinal baselines are established for different genders, age groups, races, medical conditions of the population, and the like. Computational module 106 selects an appropriate baseline for subject 102, while comparing the measured electrophysiological activity, based on the gender, age group, race, medical condition of the subject, and the like.

As mentioned earlier, different external stimuli can be used to excite different regions of the brain. The types of external stimuli that can be used to activate specific regions of brain include, but are not limited to, audio stimuli, visual stimuli, olfactory stimuli, gustatory stimuli and somato-sensory stimuli. Audio stimuli can be presented in the form of spoken words, sentences, tones, sounds, and the like. Visual stimuli can be provided by showing the subject various images, symbols, written words, sentences, and the like. Gustatory stimuli can be provided through flavors such as sweet, salty, sour, bitter, 'umami', and the like. Examples of somato-sensory stimuli include, but are not limited to, touch stimuli, exposure to mildly hot/cold, as well as to painfully hot/cold temperatures, and excitation of selected nerves by electrical pulses. Exposure to hot and cold temperatures can be provided by means of thermoelectric devices.

In accordance with an embodiment of the present invention, audio stimuli are provided to subject 102 in the form of specific words that activate different regions of the brain. The electrophysiological activity of the brain elicited by usage of various words as auditory stimuli has been extensively tested and tabular statistics are available for about 40,000 English words in Kucera, H., and Francis, W.N., Computational Analysis of Present-day American English, (1967), Brown University Press, Providence, RI. These words have been categorized into dichotomizations such as nouns vs. verbs, imageable vs. non-imageable, high affective impact vs. no affective impact, positive affect vs. negative affect, concrete idea vs. abstract idea, and the like. Similar analysis of words is also available in the Oxford Psycholinguistic Database, (1981), by Colheart et al.

A list of words to be used as auditory stimuli can be maintained in different categories, each category corresponding to a region of the brain. For example, visual words (words that refer to objects that are usually visually perceived) can be used to excite the visual cortex, and action words (words that refer to performable actions) could be used to excite the motor, pre-motor, and pre-frontal cortex. Simple auditory stimuli such as tones can be used to stimulate the auditory cortex. Emotional words can be used to stimulate the limbic brain regions, and so on.

The list can be maintained in different languages corresponding to different patients. The subject can also be exposed to stimuli by displaying these words visually.

In an embodiment, different specific words are played over audio devices such as headphones, earphones, speakers, and the like, to subject 102, and the electrophysiological activity of the brain is measured. This is compared with the pre-established second baseline of each word, to gauge the altered activity of the corresponding region of the brain. Altered activity in a region suggests an altered blood flow in that region. Since different branches of the cerebral vasculature supply blood to different regions of the brain, it is possible to identify the blood vessel supplying blood to the region of the brain showing altered activity.

Alternatively, in another embodiment of the present invention, subject 102 is instructed to perform specific tasks while the electrophysiological activity is being measured. Certain specific tasks have been used by researchers to excite specific regions of the brain while conducting the functional imaging of the brain by using functional Magnetic Resonance Imaging (fMRI) or functional Positron Emission Tomography (fPET). The correlation between the tasks performed by the subject and the regions of the brain that are excited while performing the task can be established. The specific tasks can either be active tasks or passive tasks. Various examples of passive tasks include, but are not limited to, passive listening to words and/or sentences, viewing images, and the like. Various examples of active tasks include, but are not limited to, following commands with finger, hand, limb or eye movements, responding to external stimuli, and the like.

The region of the brain with altered activity can be identified, based on the external stimuli and/or the specific task which resulted in the altered activity. The location of the region with altered activity can then be verified by using inversion algorithms on the measured electrophysiological activity as known in the art. Briefly, inversion algorithms employ a mathematical model of the brain to estimate the location of the source of the altered electrophysiological activity, based on the electrophysiological activity measured in different regions of the brain. This is referred to as the inverse problem. The inverse problem does not have a unique solution because the measured activity can result from activation of several possible brain regions. The inversion algorithm tries to predict the most likely solution. One of the ways of estimating the solution is through successive refinement. Initially, a certain region of the brain is assumed to be activated. The mathematical model of the brain is then used to compute the electrophysiological activity that would result from the current region of activation. The computed electrophysiological activity is then compared with the measured activity, and the location of assumed region of activation is then adjusted to reduce the difference between this computed activity and the measured activity. This process is iterated until convergence.

Once the region of altered electrophysiological activity is verified, the specific branches of the cerebral vasculature with an altered blood flow can be determined. Since each region of the brain receives blood through specific branches of the cerebral vasculature, any alteration in the activity of a particular region signifies that the specific branches that supply blood to that particular region have an altered blood flow.

Alerting module 108 generates an alert indicating the specific branches of the cerebral vasculature with the altered blood flow, based on information received from computational module 106.

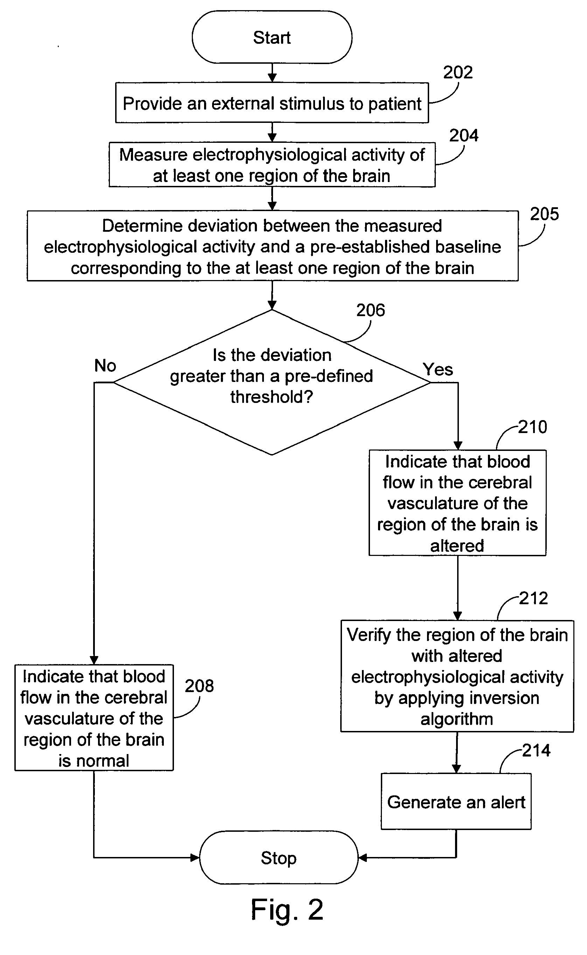

FIG. 2 is a flowchart depicting a method for monitoring the blood flow in different regions of the cerebral vasculature, according to an embodiment of the present invention.

At step 202, the subject is exposed to an external stimulus. This external stimulus can be auditory, visual, somato-sensory, gustatory or olfactory in nature (as described in detail in conjunction with FIG. 1). The external stimulus is selected based on the region of the brain to be excited.

At step 204, the electrophysiological activity of at least one region of the brain is measured.

At step 205, the deviation between the measured electrophysiological activity and a pre-established baseline corresponding to the at least one region of the brain is determined.

At step 206, the deviation is compared with a pre-defined threshold. If at step 206, the deviation is not greater than the pre-defined threshold, then at step 208, the blood flow in the region of the cerebral vasculature is indicated as normal.

However, if at step 206, the deviation is greater than the pre-defined threshold, then at step 210, the blood flow in the region of the cerebral vasculature is indicated to be altered. At step 212, the region of the brain with the altered electrophysiological activity, and in turn, the region of the cerebral vasculature with the altered blood flow, is verified through an inversion algorithm that is applied to the measured electrophysiological activity. At step 214, an alert is generated that indicates the specific branches of the cerebral vasculature with the altered blood flow.

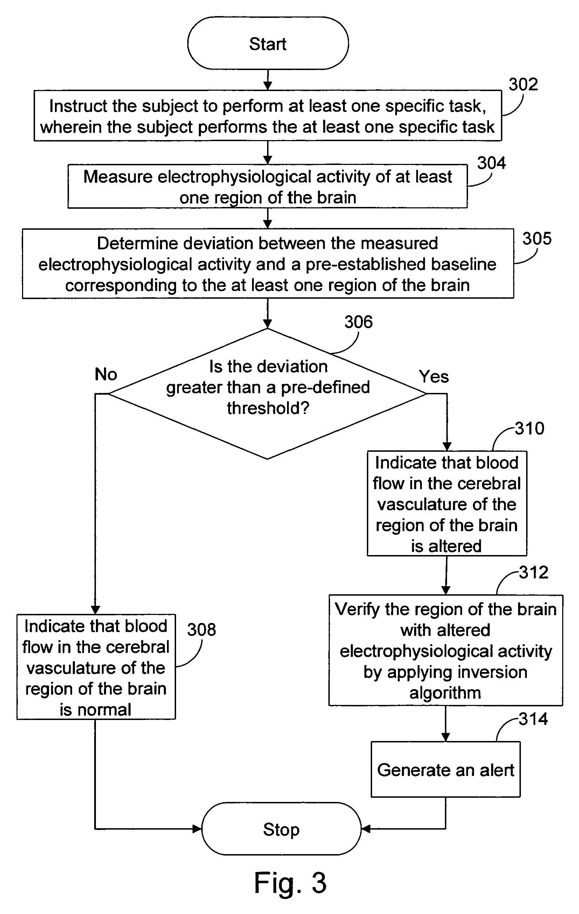

FIG. 3 is a flowchart depicting a method for monitoring the blood flow in different regions of the cerebral vasculature, according to another embodiment of the present invention.

At step 302, the subject is instructed to perform a task. Thereafter, the task is performed by the subject. This task can be an active or a passive one, and is selected based on the region of the brain to be excited, which corresponds to the region of the cerebral vasculature to be monitored.

At step 304, the electrophysiological activity of at least one region of the brain is measured.

At step 305, the deviation between the measured electrophysiological activity and a pre-established baseline corresponding to the at least one region of the brain is determined

At step 306, the deviation is compared with a pre-defined threshold. If at step 306, the deviation is not greater than the pre-defined threshold, then at step 308, the blood flow in the region of the cerebral vasculature is indicated as normal.

However, if at step 306, the deviation is greater than the pre-defined threshold, then at step 310, the blood flow in the region of the cerebral vasculature is indicated to be altered. At step 312, the region of the brain with the altered electrophysiological activity, and in turn, the region of the cerebral vasculature with the altered blood flow, is verified by an inversion algorithm, which is applied on the measured electrophysiological activity. At step 314, an alert is generated, which indicates the specific branches of the cerebral vasculature with the altered blood flow.

The method can be used to assess the distributed components of the cortical arterial system of a human body, including but not limited to the major vessels and main subdivisions forming this system, which arise as terminal branches of the anterior, middle,and posterior cerebral arteries. Examples of these arteries include but are not limited to the internal carotid artery, the vertebral artery, the basilar artery, the anterior cerebral artery, the branches of the anterior cerebral artery, the anterior communicating artery, the middle cerebral artery, the branches of the middle cerebral artery, the posterior cerebral artery, the branches of the posterior cerebral artery, the posterior communicating artery, and the branches of the posterior communicating artery. The branches of the anterior cerebral artery include the antero-medial ganglionic, anterior, posterior, inferior and middle arteries. The branches of the middle cerebral artery include the antero-lateral ganglionic, ascending parietal, inferior lateral frontal, parietotemporal, ascending frontal, temporal, internal striate, external striate, inferior lateral frontal, ascending frontal, ascending, parietotemporal, and temporal arteries. The branches of the posterior cerebral artery include the postero-medial ganglionic, posterior choroidal, postero-lateral ganglionic, anterior temporal, posterior temporal, calcarine, and parietoöccipital arteries. The branches of the posterior communicating artery include the postero-medial ganglionic, and anterior choroidal arteries.

The method can be used to monitor the blood flow in the cerebral vasculature during critical conditions, including but not limited to secondary incidences post aneurism, post-trauma monitoring, sub-arachnoid hemorrhage (vasospasm), meningitis (bacterial, viral or fungal), epilepsy, hypoxic injury, adult respiratory distress syndrome (ARDS), hypertensive encephalopathy, any acquired acute insult to the brain that might require ICU admission and observation, and the like. The method can also be used to monitor the blood flow in the cerebral vasculature during non-critical conditions, including but not limited to general bedside monitoring, during clinical trials, and for experimental purposes. Further, the method can be used in operative as well as non-operative environments.

The method and system of the present invention or any of its components may be embodied in the form of a computer system. Typical examples of a computer system include a general-purpose computer, a programmed microprocessor, a micro-controller, a peripheral integrated circuit element, and other devices or arrangements of devices that are capable of implementing the steps constituting the method of the present invention.

The computer system comprises a computer, an input device, a display unit and the Internet. The computer comprises a microprocessor, which is connected to a communication bus. The computer also includes a memory, which may include Random Access Memory (RAM) and Read Only Memory (ROM). Further, the computer system is connected to a storage device, which can be a hard disk or a removable storage such as a floppy disk, optical disk, flash card, magnetic tape, etc. The storage device can be other similar means for loading computer programs or other instructions on the computer system and can either be directly or remotely connected to the computer system. The computer system also includes a communication unit, which enables the computer to connect to other databases and the Internet through an I/O interface. The communication unit enables the transfer and reception of data from other databases, and may include a modem, an Ethernet card, or any other similar device that enables the computer system to connect to databases and networks such as LAN, MAN, WAN, and the Internet. The computer system facilitates inputs from users through an input device that is accessible to the system through an I/O interface.

The computer system executes a set of instructions that are stored in one or more storage elements, to process input data. The storage elements may hold data or other information, as desired, and may also be in the form of an information source or a physical memory element present in the processing machine.

The set of instructions may include various commands that instruct the processing machine to perform specific tasks such as the steps constituting the method of the present invention. The set of instructions may be in the form of a software program. Further, the software may be in the form of a collection of separate programs, a program module with a larger program, or a portion of a program module, as in the present invention. The software may also include modular programming in the form of object-oriented programming. Processing of input data by the processing machine may be in response to user commands, the result of previous processing, or a request made by another processing machine.

While various embodiments of the present invention have been illustrated and described, it will be clear that the present invention is not limited to these embodiments only. Numerous modifications, changes, variations, substitutions and equivalents will be apparent to those skilled in the art, without departing from the spirit and scope of the present invention, as described in the claims.

## Claims

1. A method for monitoring blood flow in at least one region of cerebral vasculature of a subject, the method comprising:
a. determining an electrophysiological activity in the at least one region of the cerebral vasculature using a non-invasive technique;
b. determining a deviation of the electrophysiological activity from a baseline of electrophysiological activity attributed to the at least one region of the cerebral vasculature; and
c. determining the blood flow in specific branches of the at least one region of the cerebral vasculature based on the deviation.

2. The method according to claim 1, further comprising providing at least one external stimulus to the subject.

3. The method according to claim 2, wherein the external stimulus is an auditory or visual stimulus.

4. The method according to claim 3, wherein providing the auditory stimulus comprises:
a. selecting at least one word from a predetermined set of words, the at least one word being selected based on the at least one region of the cerebral vasculature; and
b. audibly presenting the at least one word to the subject.

5. The method according to claim 4 further comprising maintaining a list of pre-selected words to be used for the auditory stimulus.

6. The method according to claim 3, wherein providing the visual stimulus comprises:
a. selecting at least one word from a predetermined set of words, the at least one word being selected, based on the at least one region of the cerebral vasculature; and
b. visually presenting the at least one word to the subject.

7. A method according to claim 1 further comprising, as an initial step, instructing the subject to perform at least one specific task, wherein the subject performs the at least one specific task

8. The method according to one of the preceding claims, further comprising determining a baseline of electrophysiological activity related to the at least one region of the cerebral vasculature, in particular in response to an external stimulus.

9. The method according to one of the preceding claims, further comprising detecting altered electrophysiological activity attributed to the at least one region of the cerebral vasculature when the deviation exceeds a pre-defined threshold.

10. The method according to claim 9, further comprising generating an alert.

11. The method according to claim 9 or 10 further comprising locating at least one region of the altered electrophysiological activity through application of an inversion algorithm on the electrophysiological activity.

12. The method according to one of claims 9 to 11, further comprising:
a. providing at least one external stimulus to the subject; and
b. locating at least one source of the altered electrophysiological activity by identifying the external stimulus that resulted in the altered electrophysiological activity.

13. The method according to claim 12, further comprising verifying the region of the altered electrophysiological activity through application of an inversion algorithm on the measured electrophysiological activity.

14. A computer program product for use with a computer stored program, the computer program product comprising a computer readable medium having a computer readable program code embodied therein for monitoring blood flow in at least one region of cerebral vasculature of a subject, the computer readable program code including instructions for carrying out the method of one of the preceding claims.
